# EUROPÄISCHE PATENTSCHRIFT

(11) **EP 1 853 210 B1**
(45) Veröffentlichungstag und Bekanntmachung des Hinweises auf die Patenterteilung: **09.01.2019**
(21) Anmeldenummer: 06723112.6
(22) Anmeldetag: 24.02.2006
(51) Int. Cl.: A61F 13/02

(54) **FOLIENVERBAND MIT VERBESSERTER APPLIKATIONSHILFE**
FILM STRUCTURE WITH IMPROVED APPLICATION ASSISTANCE
ASSEMBLAGE DE FILM A DISPOSITIF D'AIDE D'APPLICATION AMELIORE

(30) Priorität: 03.03.2005 DE 102005009634
(43) Veröffentlichungstag der Anmeldung: 14.11.2007
(73) Patentinhaber: Paul Hartmann AG, 89522 Heidenheim (DE)
(72) Erfinder: EFFING, Jochem, 65779 Kelkheim-Fischbach (DE); ECKSTEIN, Axel, 89522 Heidenheim (DE)
(86) Internationale Anmeldenummer: PCT/EP2006/001741
(87) Internationale Veröffentlichungsnummer: WO 2006/092248

(56) Entgegenhaltungen:
- EP-A- 0 401 949
- EP-A- 0 630 628
- DE-A1- 4 314 834
- FR-A- 2 711 056
- US-A- 4 372 303
- US-A- 4 600 001
- US-A- 5 733 251

## Beschreibung

Die Erfindung betrifft einen Folienverband mit einem Polymerfilm und einem Applikationssystem zur leichteren Handhabung des Folienverbandes.

Applikationshilfen für Pflaster oder Wundauflagen sind seit geraumer Zeit bekannt. Insbesondere kommen diese Applikationshilfen bei Film- oder Folienverbänden zum Einsatz. Folienverbände sind dünne, meist transparente, semipermeable Filme oder Folien aus Polymermaterialien. Die Semipermeabilität der Folien verhindert das Eindringen von Bakterien oder Nässe und gestattet dabei einen ausreichenden Sauerstoff- und Wasserdampfaustausch zwischen der abzudeckenden Haut und der äußeren Umgebung des Folienverbandes. Diese Film- oder Folienverbände kommen in vielfältiger Weise zum Einsatz zum Beispiel als Inzisionsfolie zur keimfreien Abdeckung von Operationsstellen, als wasserdichte Abdeckung von Exsudat aufsaugenden Wundauflagen sowie zur Fixierung von Kanülen oder Kathetern. Aufgrund der geringen Dicken dieser Filme oder Folien und der damit verbundenen Instabilität, werden diese Folienverbände mit den unterschiedlichsten Applikationshilfen ausgestattet. Die meisten dieser Applikationshilfen bedienen sich einer zusätzlichen Stützschicht, die während oder nach der Applikation des Folienverbandes entfernt werden.

Filmwundauflagen sind seit geraumer Zeit auch in der Patentliteratur bekannt. So beschreibt die EP 81 990 B1 eine adhäsive Wundauflage, die aus einem dünnen Polymerfilm besteht. Dieser Polymerfilm ist auf einer ersten Seite mit einem auf der Haut klebenden Haftklebstoff beschichtet, der seinerseits mit einer Ablöseschicht abgedeckt ist. Auf der anderen, in Gebrauch körperabgewandten Seite weist der Polymerfilm zur leichteren Handhabung zudem eine leicht zu entfernende Stützschicht aus einem Fasermaterial zum Beispiel ein undurchsichtiges Nonwoven auf. Diese Stützschicht weist dieselbe Größe wie der Polymerfilm auf.

Mit EP 690 706 B1 wird ein klebender Wundverband beschrieben, der zur leichteren Applikation eines Polymerfilms, der von dem Wundverband umfasst ist, eine Trägerschicht aufweist. Diese Trägerschicht deckt vollflächig den Polymerfilm ab und kann in zwei Schritten von dem Polymerfilm entfernt werden. Hierzu wird im ersten Schritt ein Mittelteil aus der Trägerschicht entfernt, worauf im zweiten Schritt ein Rahmenteil entfernt wird. Nachteilig ist, dass die Trägerschicht dieses Wundverbandes nur sehr schwer vom Benutzer ergriffen werden kann.

Des Weiteren beschreibt die EP 951 263 B1 einen klebenden Folienverband, dessen klebende Seite mit einer mindestens zweiteiligen abziehbaren Schutzschicht abgedeckt ist und dessen nicht klebende zweite Seite vollflächig mit einer einteiligen Stützschicht ausgestattet ist. Bei diesem Folienverband ist die Stützschicht mit der Schutzschicht an zwei gegenüberliegenden Seiten scharnierartig verbunden, so dass mit dem Entfernen der Schutzschicht gleichzeitig die Stützschicht entfernt werden kann.

Mit der EP 473 918 B1 wird ein Folienverband beschrieben, der eine einteilige Stützfolie aufweist, die ihrerseits an zwei gegenüberliegenden Seiten jeweils eine Griffleiste aufweist. Durch diese Anordnung der Griffleisten, ergibt sich der Nachteil, dass keine Abzugsrichtung für die Stützfolie vorgegeben ist.

Die EP 985 931 A1 beschreibt ein Verbandmaterial auf Folienbasis, das im Randbereich der Folie einen nicht klebenden Anfasser aufweist. Die nicht klebende Seite der Folie ist mit einer einteiligen Stützschicht versehen, die die gleiche Größe wie die Folie aufweist und mindestens eine Griffleiste aufweist. Durch Ziehen an dem Anfasser in Verklebungsrichtung kann die applizierte Folie wieder schmerzfrei entfernt werden.

Durch die europäische Patentschrift EP 630 628 B1 ist ein Filmverband bekannt, der zur leichteren Applikation eine zweigeteilte Stützfolie umfasst. Diese Stützfolie ist größer als der zu applizierende Film und bedeckt diesen vollflächig. Zum Entfernen der Stützfolie ist auf der Stützfolie ein weiterer adhäsiv ausgerüsteter Abziehstreifen angebracht, der über der Schnittlinie der Stützfolie angeordnet ist und zum Ergreifen zwei nicht klebende Randbereiche aufweist, die als Griffleiste dienen. Mit Hilfe dieses weiteren Abziehstreifens kann lediglich ein Teil der Stützfolie entfernt werden, worauf der zweite Teil der Stützfolie auf dem Polymerfilm verbleibt.

Mit der WO 97/25012 A1 wird ein Folienverband vorgeschlagen, der vollflächig oder lediglich an zwei gegenüberliegenden Randbereichen der Folie mit einer zweiteiligen Stützschicht versehen ist. Falls die Stützschicht vollflächig auf den Folienverband aufgebracht wird, so können auf der Stützfolie Anfasshilfen aufgebracht sein. Die der Stützschicht gegenüberliegende adhäsive Schutzschicht ist in drei Teile unterteilt.

Mit der europäischen Anmeldung EP-401949-A2 wird ein klebender Dünnfilm mit einem Applikationssystem für den Film beschrieben. Das Applikationssystem besteht aus zwei Stützfolien, die mittels eines Klebemittels auf der körperabgewandten Seite des Films aufgebracht sind und die an zwei Endbereichen zum leichteren Ergreifen und Entfernen von dem Film frei von dem Klebemittel ausgebildet sind. Hierbei überfängt ein erster Bereich einer ersten Stützfolie einen ersten Bereich einer zweiten Stützfolie.

Mit der französischen Patentanmeldung FR-2711056-A1 wird ein absorbierender Wundverband beschrieben, der aus einem dünnen Film, einem Wundkissen und einer Applikationsfolie zur leichteren Applikation des Wundverbandes besteht. Der Wundverband umfasst weiterhin Perforationslinien, mittels derer definierte Bereiche des Films oder der Folie von zu applizierenden Bereichen des Films getrennt werden können.

Mit der US-amerikanischen Anmeldung US-2004/0162512-A1 ist ein Wundverband bekannt, der eine Trägerschicht aus einem dünnen Polymerfilm und ein radiales Applikationssystem zur leichteren Applikation des Wundverbandes umfasst. Das Applikationssystem ist derart gestaltet, dass der Polymerfilm alternierend durch das Applikationssystem abgedeckte und nicht abgedeckte Bereiche aufweist.

Mit der US-amerikanischen Patentschrift US-4372303-A wird ein System zur Applikation von Bandagen, Abdeckungen und Wundauflagen beschrieben. Das System besteht aus einem dünnen auf dem Körper eines Patienten zu applizierenden Film, der auf der dem Patienten abgewandten Seite zur leichteren Applikation mit einem Rahmen abgedeckt ist. Der Rahmen kann vollflächig sein oder mit einer zentrisch liegenden Öffnung versehen sein. Mit der Patentschrift ist nicht bekannt, den Rahmen zweiteilig zu gestallten und jeden Teil mit einer Griffleiste zu versehen. Darüber hinaus ist nicht bekannt, dass eine angeformte Griffleiste einen ersten stützfolienfreien Bereich überfängt.

Diese Schutzrechte zeigen insgesamt verschiedene alternative Lösungen zu Film- oder Folienverbänden mit verschiedenen Applikationssystemen auf. Teilweise werden die mit diesen Schutzrechten als Lösung vorgeschlagenen Folienverbände als zu kompliziert im Aufbau und zu kompliziert in der Anwendung angesehen. Darüber hinaus weisen die mit diesen Schutzrechten vorgeschlagenen Folienverbände mit Applikationshilfen insgesamt eine Steifigkeit auf, die entgegen dem eigentlich zu applizierenden sehr flexiblen Polymerfilm als zu hoch empfunden wird. Diese Flexibilität der Folienverbände ist jedoch erforderlich, um die eigentlich zu applizierenden Polymerfilme zielgenau und faltenfrei zu applizieren.

Aufgabe der vorliegenden Erfindung ist es somit, einen Folienverband mit einem Applikationssystem zur Verfügung zu stellen, der einen einfachen Aufbau aufweist und dennoch eine faltenfreie Applikation des Polymerfilms gewährleistet. Darüber hinaus besteht eine Aufgabe darin, einen Folienverband zur Verfügung zu stellen, der handhabungssicher und gezielt auf die vorgesehene Fläche aufgebracht werden kann. Dabei soll der Folienverband ohne jede Form- oder Größeneinschränkung universell einsetzbar sein.

Gelöst wird diese Aufgabe durch einen Folienverband gemäß den Merkmalen des Anspruchs 1. Demnach umfasst der Folienverband einen Polymerfilm und zur leichteren Handhabung des Folienverbandes ein Applikationssystem, wobei das Applikationssystem auf einer ersten Seite des Polymerfilms angeordnet ist und mindestens eine Stützfolie umfasst, an der mindestens eine Griffleiste angeformt ist. Das Applikationssystem umfasst dabei erfindungsgemäß zwei Stützfolien mit jeweils einer Griffleiste, wobei jede Griffleiste eine zusätzliche Materialkomponente ist und gleichzeitig mit mindestens einer Griffleiste von dem Polymerfilm entfernbar ist. Der Polymerfilm weist weiterhin mindestens einen ersten stützfolienfreien Bereich auf wobei eine Griffleiste den ersten stützfolienfreien Bereich zumindest abschnittsweise überfängt und wobei die Auflagefläche der Stützfolien insgesamt weniger als 94 % der Fläche der ersten Seite des zu applizierenden Polymerfims entspricht.

Ein Vorteil eines solchen Folienverbands mit Applikationssystem besteht darin, dass der stützfolienfreie Bereich des Polymerfilms, das heißt der nicht durch eine Stützfolie abgedeckte Bereich, aufgrund der leichteren Biegsamkeit im Vergleich zum Polymerfilm mit Stützfolie, während der Applikation des Folienverbandes als Gelenk fungieren kann. Somit kann als Stützfolie auch ein relativ steifes Stützmaterial zum Einsatz gebracht werden, wobei gleichzeitig eine passgenaue Applikation gewährleistet ist. Beim Stand der Technik mit einer einteiligen Stützfolie dagegen, die gleich groß wie der Polymerfilm ist oder bei einer mehrteiligen Stützfolie, die insgesamt gleich groß wie der Polymerfilm ist, muss die Wahl des Stützmaterials auf relativ flexible Materialien eingeschränkt werden, um eine passgenaue Applikation des Polymerfilms zu gewährleisten. Ein weiterer Vorteil besteht in der Materialeinsparung der für die Stützfolien verwendeten Materialien.

Gleichwohl verringert die zumindest abschnittsweise Abdeckung des stützfolienfreien Bereiches durch die Griffleiste die Gefahr der Verunreinigung oder Beschädigung des Polymerfilmes.

Als Applikationssystem soll bei einer erfindungsgemäßen Ausführung alles verstanden sein, das der leichteren Applikation des Polymerfilms dienen kann und mindestens eine Stützfolie und zusätzlich zu dieser Stützfolie zumindest eine Griffleiste umfasst, die an dieser Stützfolie angeformt ist. Unter Anformung soll hier das Verbinden zweier gleicher oder zweier verschiedener Materialien verstanden sein, die mit Hilfe von Klebemitteln, Druck, thermischer Energie, Ultraschallverfahren oder sonstiger Verfahren miteinander lösbar oder unlösbar verbunden sind. Die Griffleiste ist also vorliegend immer eine zusätzliche Materialkomponente, wobei die Griffleiste immer gleichzeitig mit mindestens einer Stützfolie von einem Polymerfilm entfernbar ist. Des Weiteren soll des leichteren Verständnisses wegen im Zusammenhang mit der vorliegenden Erfindung unter einem Film oder Polymerfilm immer der eigentlich zu applizierende Film oder Polymerfilm wie zum Beispiel ein Wundverband verstanden sein; dagegen soll unter einer Folie oder Polymer- bzw. Stützfolie immer ein Teil des Applikationssystems verstanden sein, das heißt die Unterscheidung zwischen Film und Folie ist hier lediglich auf die Funktion der Bestandteile zu verstehen. Hinsichtlich des Materials soll, wie im Sprachgebrauch üblich, kein Unterschied zwischen einem Film und einer Folie verstanden sein.

Durch den ersten stützfolienfreien Bereich ist die durch die Stützfolien abgedeckte Fläche des Polymerfilms somit kleiner ist als die Fläche der ersten Seite des Polymerfilms.

In einer weiteren Ausgestaltung der Erfindung ist vorgesehen, dass eine Griffleiste einen nicht durch die Stützfolien abgedeckten Bereich des Polymerfilmes vollständig überfängt. In diesem Fall kann das Applikationssystem insgesamt dieselbe Größe wie der Polymerfilm aufweisen. Hierbei ist mit derselben Größe, eine Größe hinsichtlich der Auflagefläche gemeint, das heißt die Umfangsbegrenzungen des Applikationssystems und des Polymerfilmes sind bündig. Durch dieselbe Größe des Applikationssystems und des Polymerfilms und dadurch, dass die Griffleiste lediglich an der Stützfolie angeformt ist und keine Verbindung zu dem Polymerfilm aufweist, ist gewährleistet, dass neben der bereits geschilderten hohen Flexibilität in dem nicht durch die Stützfolie abgedeckten Bereich zudem der gesamte Film abgedeckt ist und somit auch vor und während der Applikation vollständig geschützt ist.

In einer bevorzugten Ausführungsform eines erfindungsgemäßen Folienverbandes ist vorgesehen, dass eine Griffleiste eine zum Ergreifen durch den Benutzer bestimmte Grifffläche, vorzugsweise ausgeführt als Hintergriffsmittel, von mindestens 2 cm², insbesondere mindestens 5 cm² und ganz besonders bevorzugt von mindestens 7 cm² aufweist.

Insbesondere kann ein erster stützfolienfreier Bereich an einem Rand des Polymerfilms angeordnet sein. Als Rand soll hier jeder Bereich einer Fläche verstanden sein, der sich von der Kante einer Fläche in das Innere einer Fläche erstreckt, wobei die flächenhafte Ausdehnung des Randes kleiner ist als 50% der gesamten Fläche. Hierdurch wird ein Folienverband bereitgestellt, der vorteilhafter Weise einen Bereich aufweist, der eine durch den Polymerfilm selbst vorgegebene Flexibilität aufweist und eine leichte Erstfixierung des zu applizierenden Films gewährleistet, wobei gleichzeitig durch die Stützfolien auf den weiteren Bereichen eine sichere Handhabung gewährleistet ist. Hierbei hat sich als besonders gut handhabbar und anwendungssicher herausgestellt, wenn eine der Stützfolien in mindestens einem Punkt ihrer äußeren Kante einen Abstand von der äußeren Kante des Polymerfilms von mindestens 2 mm, insbesondere mindestens 3 mm und ganz besonders mindestens 5 mm besitzt. Besonders bevorzugt ist ein Abstand, der in jedem Punkt der Kante der Stützfolie einen gleichen Abstand von mindestens 2 mm, insbesondere mindestens 3 mm und ganz besonders mindestens 5 mm zu der äußeren Kante des Polymerfilms aufweist.

In einer weiteren Ausführung dieser Erfindung umfasst das Applikationssystem zwei Stützfolien, die in einer Ebene parallel zu dem Polymerfilm aufgebracht werden. In einer solchen Ausgestaltung der Erfindung kann ein erster stützfolienfreier Bereich zwischen der ersten und der zweiten Stützfolie liegen. Der Abstand dieser beiden Stützfolien zueinander beträgt dabei vorzugsweise in jedem Punkt mindestens 2 mm, insbesondere 3 mm und ganz besonders 5 mm. Besonders bevorzugt ist ein Applikationssystem, das zwei Stützfolien aufweist, die in jedem Punkt den gleichen Abstand zueinander aufweisen.

Insbesondere ist bei der Ausgestaltung des Folienverbandes mit zwei Stützfolien vorgesehen, dass jede Stützfolie jeweils eine Griffleiste umfasst. Dabei werden eine erste Griffleiste der ersten Stützfolie und eine zweite Griffleiste der zweiten Stützfolie zugeordnet.

In einer bevorzugten Ausführungsform dieses erfindungsgemäßen Folienverbandes mit zwei Stützfolien ist vorgesehen, dass die erste Griffleiste eine zum Ergreifen durch den Benutzer bestimmte Grifffläche, vorzugsweise ausgeführt als Hintergriffsmittel, aufweist und die erste Griffleiste mit dieser Grifffläche die zweite Griffleiste zumindest abschnittsweise, insbesondere aber vollständig überfängt.

Durch diese Anordnung der Griffleisten ist in besonders einfacher Weise gewährleistet, dass der Anwender in jedem Fall nur die zu oberst liegende erste Griffleiste als erste Griffleiste betätigen kann und somit eine erste Stützfolie als erste Folie von dem Polymerfilm entfernen kann. Erst im zweiten Schritt kann der Anwender daraufhin eine zweite Stützfolie mit Hilfe der zweiten Griffleiste entfernen. Es ist somit eine Reihenfolge bei der Entfernung der Stützfolien vorgegeben und ein besonders handhabungssicherer Folienverband bereitgestellt.

Vorzugsweise beträgt die Größe der Grifffläche der ersten Griffleiste mindestens 2 cm², insbesondere mindestens 5 cm² und ganz besonders bevorzugt von mindestens 7 cm². Als besonders handhabungssicher hat sich herausgestellt, wenn die erste Griffleiste eine freie Grifffläche von mindestens 2 cm², insbesondere 4 cm² und ganz besonders bevorzugt von 6 cm² aufweist. Diese freie Grifffläche ist vorliegend der Teil der Grifffläche, der über die zweite Griffleiste seitlich übersteht.

Als besonders handhabungssicher hat sich dabei herausgestellt, wenn die erste Griffleiste sowohl den stützfolienfreien Bereich des Polymerfilms als auch die zweite Griffleiste vollständig überfängt.

Falls der Folienverband ein Applikationssystem mit zwei Stützfolien aufweist und ein erster stützfolienfreier Bereich zwischen den Stützfolien vorgesehen ist, kann getrennt von diesem ersten stützfolienfreier Bereich ein zweiter stützfolienfreier Bereich vorgesehen sein. Dieser zweite Bereich kann weiterhin vorzugsweise durch eine Griffleiste überfangen sein. Es kann aber auch vorgesehen sein, dass dieser zweite Bereich weder von einer Stützfolie noch von einer Griffleiste überdeckt wird. Vorzugsweise ist dieser zweite stützfolienfreie Bereich des Polymerfilms an einem Rand des Folienverbandes angeordnet. Auf diese Weise weist der Folienverband sowohl ein Gelenk innerhalb des Verbandes auf als auch einen Bereich zur Erstfixierung auf.

Falls ein Applikationssystem vorgesehen ist, das zwei oder mehr als zwei Stützfolien umfasst, kann jeder Stützfolie eine Griffleiste zugeordnet sein. In einer besonders bevorzugten Ausführung kann auch eine Griffleiste zwei Stützfolien zugeordnet sein. Insbesondere kann in einem Folienverband mit drei Stützfolien eine Griffleiste zwei Stützfolien zugeordnet sein. Durch diese Anordnung bzw. Zuordnung der Griffleisten auf den Stützfolien können zwei separate Stützfolien in einem Arbeitsschritt entfernt werden.

Als Stützfolien sind besonders transparente oder transluzente Folienmaterialien vorgesehen. Alternativ können jedoch auch opake oder undurchsichtige Folienmaterialien verwendet werden. Insbesondere kommen als Stützfolie solche Folien zur Verwendung, die aus Polyester, Polyethylen, Polypropylen, Polyvinylchlorid, Polystyrol, Polyamid, Polykarbonat, Celluloseester, Ethylenvinylacetat, Polyvinylacetat, Polyvinylalkohol und/oder Mischungen hiervon gefertigt werden. Besonders bevorzugt sind Stützfolien aus transparentem Polyester oder Polyethylen oder Polypropylen. Dabei hat es sich weiterhin als besonders vorteilhaft erwiesen, wenn die Dicken der Stützfolie so eingestellt werden, das diese eine Dicke von 15 bis 80 µm, insbesondere von 20 bis 60 µm und ganz bevorzugt von 20 bis 40 µm aufweisen.

Zur Herstellung einer Griffleiste können dieselben Materialien verwendet werden, die als Stützfolie zur Anwendung gebracht werden. In einer besonders bevorzugten Ausführungsform wird die Griffleiste aber aus einem Folienmaterial hergestellt, das flexibler ist als die Stützfolie. Falls ein Applikationssystem vorgesehen ist, das zwei oder mehr Stützfolien und zwei oder mehr Griffleisten umfasst, sind vorzugsweise alle Griffleisten aus einem Material gefertigt, das flexibler ist als jede Stützfolie. Hierdurch ist gewährleistet, dass die Griffleisten besonders leicht zu ergreifen sind. In einer weiteren besonders bevorzugten Ausführungsform mit zwei Griffleisten ist vorgesehen, dass die Griffleiste der ersten Stützfolie flexibler ist als die Griffleiste der zweiten Stützfolie. Dabei ist es weiterhin vorteilhaft, wenn die zweite Griffleiste vollständig von der ersten Griffleiste übergriffen wird.

Zusätzlich kann bei einem System mit zwei Griffleisten ein Aktivierungsmittel vorgesehen sein, dass zwischen der ersten und der zweiten Griffleiste angeordnet ist. Dieses Aktivierungsmittel kann zum Beispiel ein zusätzlicher adhäsiver Streifen sein, der zu beiden Seiten seiner Auflageflächen eine unterschiedliche Klebekraft aufweist. Bei der Anwendung eines solchen Folienverbandes kann dann zum Beispiel eine über der zweiten Griffleiste angeordnete erste Griffleiste ergriffen werden und mit dieser Griffleiste das Aktivierungsmittel und eine Stützfolie von dem Polymerfilm entfernt werden, wobei gleichzeitig die zweite Griffleiste so aktiviert wird bzw. aufgerichtet wird, dass diese von dem Anwender leichter greibar ist.

Alternativ kann in einer weiteren Ausführung des Folienverbandes auch vorgesehen sein, dass lediglich ein Umfangsbereich des Polymerfilms zumindest abschnittsweise von zumindest einer Stützfolie abgedeckt ist und ein im Inneren des Umfangsbereiches des Polymerfilms angeordneter stützfolienfreier Zentrumsbereich verbleibt. In dieser Ausgestaltung ist die Stützfolie als eine Art Rahmen vorgesehen, der dem Folienverband die notwendige Stabilität und Sicherheit hinsichtlich einer faltenfreien Applikation verleiht und unabhängig von dem verwendeten Material der Stützfolie gleichzeitig ein genaues Anvisieren der zu applizierenden Stelle ermöglicht. Bei dieser Ausgestaltung können somit nicht nur transparente oder transluzente sondern auch opake bzw. undurchsichtige Stützfolien zur Verwendung gebracht werden. Die auf der Stützfolie aufgebrachte Griffleiste ist vorzugsweise aus einem transparenten oder transluzenten Material gefertigt.

Als Polymerfilm können in einem erfindungsgemäßen Folienverband insbesondere Polymerfilme eingesetzt werden, die eine hohe Wasserdampfdurchlässigkeit aufweisen. Hierzu sind besonders Filme geeignet, die aus Polyurethan, Polyetherurethan, Polyesterurethan, Polyether-Polyamid-Copolymeren, Polyacrylat oder Polymethacrylat gefertigt werden. Insbesondere ist als Polymerfilm ein Polyurethanfilm, Polyesterurethanfilm oder Polyetherurethanfilm bevorzugt. Ganz besonders sind aber auch solche Polymerfilme bevorzugt, die eine Dicke von 15 bis 50 µm, insbesondere 20 bis 40 µm und ganz besonders bevorzugt von 25 bis 30 µm aufweisen. Die Wasserdampfdurchlässigkeit des Polymerfilms in einem erfindungsgemäßen Folienverbande weist vorzugsweise mindestens 750 g/ m²/ 24 Std., insbesondere mindestens 1000 g/ m²/ 24 Std. und ganz besonders bevorzugt mindestens 2000 g/ m²/ 24 Std. auf (gemessen nach DIN 13726).

Auf der dem Applikationssystem gegenüberliegenden zweiten Seite des zu applizierenden Polymerfilms kann ein Klebemittel aufgebracht sein. Dieser Auftrag kann sowohl vollflächig als auch diskontinuierlich oder nur in bestimmten Bereichen erfolgen. Bei dem verwendeten Klebemittel kann es sich um einen üblichen Haftkleber, insbesondere um einen Acrylathaftkleber oder einen druckempfindlichen Haftkleber auf Basis von Polyurethanen handeln. Bevorzugt handelt es sich um Gelhaftkleber, insbesondere auf Basis von Polyurethanen, insbesondere wässrigen Polyurethanen. Ganz besonders bevorzugt handelt es sich um Hydrogelhaftkleber, insbesondere auf Basis von wässrigen Acrylaten.

Vorteilhafterweise beträgt das Flächengewicht des Klebemittels 20-100g/m², insbesondere 30-50 g/m², wobei das Klebemittel diskontinuierlich, vorzugsweise aber vollflächig aufgetragen sein kann.

Die Wasserdampfdurchlässigkeit des mit dem Klebemittel versehenen Polymerfilm beträgt vorzugsweise mindestens 1000 g/ m²/ 24 Std., besonders bevorzugt mindestens 1200 g/ m²/ 24 Std. und ganz besonders bevorzugt mindestens 2000 g/ m²/ 24 Std. (gemessen nach DIN EN 13726) aufweisen.

Gemäß einer Weiterführung der Erfindung kann der Folienverband auf der dem Applikationssystem gegenüberliegenden zweiten Seite des Polymerfilms vollflächig mit einem Haftklebemittel beschichtet sein und das Haftklebemittel mit einer Abdeckfolie oder einem Abdeckpapier geschützt sein. Als Abdeckschicht kann dabei jedes handelsübliche Silikonpapier oder -folie sowie eine mit einer Fluorverbindung beaufschlagtes Papier oder Folie verwendet werden.

Falls der Folienverband als Wundauflage gefertigt werden soll, ist gemäß einer weiteren Ausführungsform vorgesehen ein Wundpad oder Wundkissen auf der dem Applikationssystem gegenüberliegenden zweiten Seite des Polymerfilms anzuordnen. Ein solcher Folienverband ist besonders als Wundauflage geeignet, wenn das Wundpad oder -kissen adhäsiv mit dem Polymerfilm verbunden ist. Dieses Wundkissen kann aus einem Vlies also einem Nonwoven gefertigt sein. Vorzugsweise kann es sich bei dem Vlies um ein hydrophiles Fasermaterial wie zum Beispiel Baumwolle, Viskose, Cellulose und Polyester oder deren Mischungen mit vorzugsweise hydrophilisiertem Polyethylen oder Polypropylen handeln.

Insbesondere kann an Stelle des Wundkissens oder zusätzlich zum Wundkissen der Folienverband auf der dem Applikationssystem gegenüberliegenden zweiten Seite des Polymerfilms eine, die Wundheilung fördernde Schicht aufgebracht sein. Unter einer wundheilungsfördernden Schicht soll hierbei jede Schicht verstanden sein, die zur Anwendung in der feuchten Wundbehandlung zu verwenden ist. Insbesondere sind hierzu Hydrogele auf Basis von Polyurethanen, Acrylaten oder wasserlöslichen Cellulosen oder Mischungen hiervon bevorzugt, die einen Wasseranteil von mindestens 20 %, insbesondere mindestens 50% bezogen auf das Gesamtgewicht des Hydrogels aufweisen. Diese Hydrogele können sowohl auf das Wundkissen als auch auf die zweite Seite des Polymerfilmes direkt aufgebracht sein.

Um einen anwendungssicheren Folienverband bereit zu stellen, muss eine genaue Abstimmung der verwendeten Materialien erfolgen. Insbesondere müssen die verwendeten Materialien hinsichtlich ihrer Release-Eigenschaften abgestimmt werden. Diese durch zusätzliche Mittel einstellbaren Release-Eigenschaften beruhen auf den Kräften, die zwischen zwei verwendeten Materialien herrschen. So kann zum Beispiel durch eine gezielte Oberflächenbehandlung eines Materials eine anziehende oder eine abstoßende Wirkung auf ein zweites mit diesem ersten Material zusammen zu bringendes Material eingestellt werden. Eine Oberflächenbehandlung, die eine anziehende Wirkung zwischen zwei Materialien hervorruft, kann zum Beispiel durch einen zusätzlichen adhäsiven Auftrag, eine statische Aufladung oder durch ein Verschmelzen der beiden zusammenzubringenden Materialien erfolgen. Eine abstoßende Wirkung kann zum Beispiel durch eine zusätzliche Schicht auf einem Material aus Silikon- oder Fluorverbindungen hervorgerufen werden. Als Release-Kraft (Abziehkraft) wird dabei eine solche Kraft verstanden, die notwendig ist zwei Materialien voneinander zu trennen (gemessen nach DIN 53530).

In einer weiteren Ausführungsform des erfindungsgemäßen Folienverbandes sind diese Release-Eigenschaften so eingestellt, dass die Abziehkraft, die notwendig ist um eine Abdeckfolie oder -papier von dem zu applizierenden Polymerfilm zu lösen, größer ist als die Abziehkraft, die notwendig ist, um die Stützfolie oder die Stützfolien von dem Polymerfilm zu trennen.

Bei einer Ausgestaltung des Folienverbandes mit zwei Stützfolien sind die Release-Eigenschaften bevorzugt so eingestellt, dass die Abziehkraft, die notwendig ist um die erste Stützfolie von dem zu applizierenden Polymerfilm zu lösen, gleich groß ist wie die Abziehkraft, die zum Lösen der zweiten Stützfolie notwendig ist.

Weiterhin bevorzugt sind bei einem Folienverband mit zwei Stützfolien und zwei Griffleisten die Release-Eigenschaften so eingestellt, das die Abziehkraft, die notwendig ist, um die erste Griffleiste von der zweiten Griffleiste oder die zweite von der ersten Griffleiste zu lösen, geringer ist als die Ablösekraft, die notwendig ist, um die Stützfolie von dem zu applizierenden Polymerfilm zu lösen.

Bei einer weiteren Ausgestaltung des Folienverbandes mit zwei Stützfolien sind die Release-Eigenschaften bevorzugt so eingestellt, das die Abziehkraft, die notwendig ist, um die erste Stützfolie von dem zu applizierenden Polymerfilm zu lösen, größer ist als die Abziehkraft, die zum Lösen der ersten Griffleiste von der zweiten Griffleiste notwendig ist.

Die Haftung der Stützfolie auf dem Polymerfilm ist vorzugsweise nur 0,01 bis 0,5 N/ 25 mm, ganz besonders bevorzugt 0,01 bis 0,1 N/ 25 mm, gemessen nach DIN 53530. Hierzu, wird das Stützmaterial vorzugsweise direkt bei der Herstellung des Polymerfilms auf den Polymerfilm aufgebracht, bzw. der Polymerfilm direkt auf dem Stützmaterial erzeugt. Hierbei können alle üblichen Verfahren zur Filmherstellung angewendet werden zum Beispiel durch Giessen, Rakeln, Extrudieren oder andere bekannte Methoden der Film- oder Folienherstellung. Falls notwendig, kann das Stützmaterial auf der Beschichtungsseite aufgeraut oder einer anderen haftungsfördernden Behandlung unterworfen werden. Auch eine adhäsionsfördernde Beschichtung kann vorteilhaft sein. Wichtig dabei ist, dass die Haftung des Polymerfilms auf der zu applizierenden Oberfläche wesentlich größer ist als die Haftung einer Stützfolie an dem Polymerfilm.

In einer besonderen Ausgestaltung der Erfindung ist vorgesehen, dass ein Folienverband umfassend einen Polymerfilm mit einem Applikationssystem in einer Verpackung angeordnet ist. Insbesondere ist dabei vorgesehen, dass die Verpackung eine sterile Verpackung ist.

Hervorzuheben ist an dieser Stelle, dass die hier aufgeführten Merkmale der alternativen Ausgestaltungen der Erfindungen nicht auf die einzelnen Alternativen zu beschränken sind. Es ist vielmehr der Fall, dass die Kombination der Ausgestaltungen bzw. die Kombination der Einzelmerkmale der alternativen Formen ebenso zu einer erfindungsgemäßen Ausgestaltung zu zählen ist. Ebenso wenig soll die Erfindung durch die nachfolgende Beschreibung der Zeichnungen reduziert verstanden sein. Nachstehend wird die Erfindung unter Bezugnahme auf die Zeichnung erläutert. In den Zeichnungen zeigt:
Figur 1: ein Folienverband (nicht Teil der vorliegenden Erfindung), in Aufsicht
Figur 2: eine Ausführungsform eines Folienverbandes, in Aufsicht
Figur 3: eine weitere Ausführungsform eines Folienverbandes, in Aufsicht
Figur 4: die in Figur 1 wiedergegebene Ausführungsform eines Folienverbandes, im Querschnitt (nicht Teil der vorliegenden Erfindung)
Figur 5: die in Figur 2 wiedergegebene Ausführungsform eines Folienverbandes, im Querschnitt
Figur 6: die in Figur 3 wiedergegebene Ausführungsform eines Folienverbandes, im Querschnitt
Figur 7 a - c: eine Ausführungsform bei der Verwendung, im Querschnitt

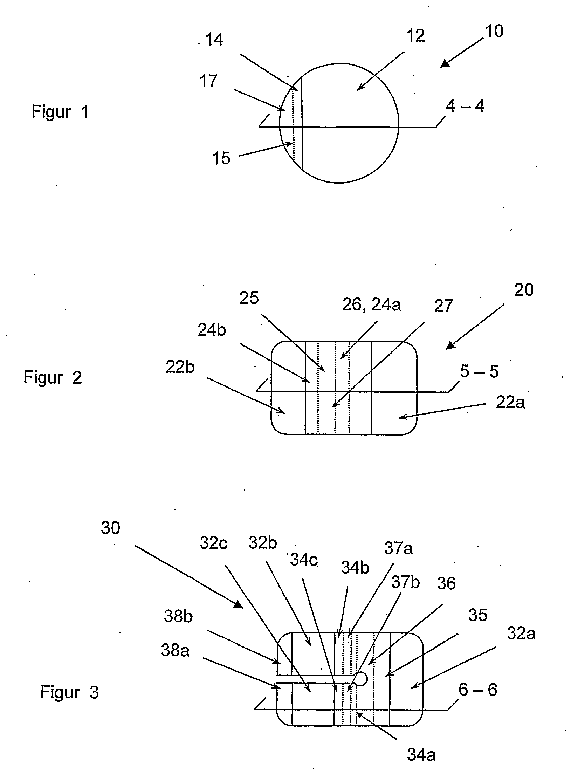

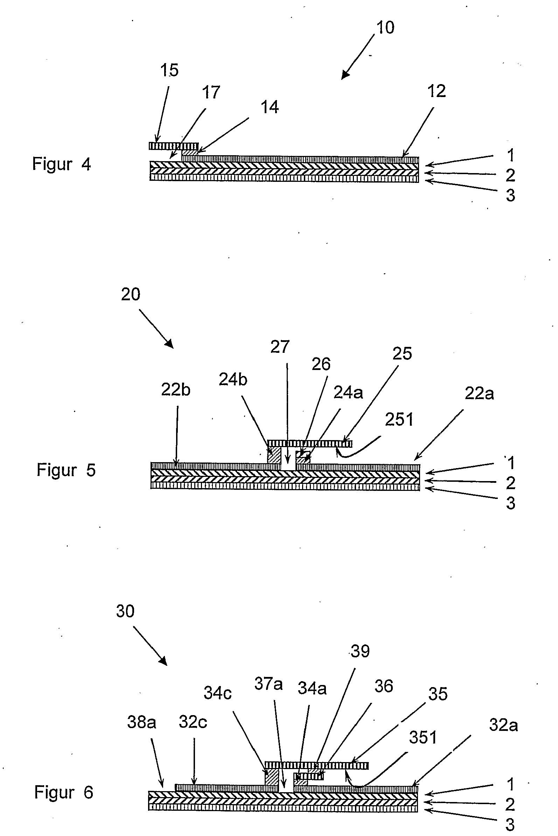

Mit Figur 1 und Figur 4 ist ein Folienverband, der nicht Teil der vorliegenden Erfindung ist, gezeigt. Der Folienverband (10) liegt als Ganzes in kreisrunder Form vor. Er besteht aus einem transparenten Polymerfilm (1) der auf seiner ersten Seite mit einem Applikationssystem abgedeckt ist. Auf der dem Applikationssystem abgewandten zweiten Seite ist ein adhäsives Klebemittel (2) aufgebracht, das durch eine Abdeckschicht (3) geschützt ist. Das Applikationssystem besteht aus einer ebenfalls transparenten Stützfolie (12), die einen Teil des Polymerfilms bedeckt, einer Griffleiste (15) und einem Adhäsiv (14). In einem Randsegment (17) ist der Polymerfilm stützfolienfrei. Auf der Stützfolie (12) ist mittels des Adhäsivs (14) die Griffleiste (15) angeformt. Diese Griffleiste überfängt vollständig den stützfolienfreien Bereich (17) des Polymerfilms. Die Griffleiste ist durch keine Mittel mit dem Polymerfilm verbunden, so dass diese bei Gebrauch ohne Aufwand sofort greifbar ist. Der Polymerfilm wird vollständig durch das Applikationssystem abgedeckt, wobei gleichzeitig durch den stützfolienfreien Bereich (17) ein flexibler Bereich geschaffen wurde der hervorragend bei der Anwendung des Folienverbandes zur Erstfixierung verwendet werden kann.

Mit Figur 2 und Figur 5 ist eine Ausführungsform der vorliegenden Erfindung gezeigt. Dieser Folienverband (20) ist von rechteckiger Grundform und besteht wie die erste Ausführung aus einem Polymerfilm (1), einem auf den Polymerfilm aufgebrachten vollflächigen Klebstoffauftrag (2) und einer diesen Klebstoff abdeckenden Abdeckschicht (3). Auf der ersten Seite des Polymerfilms weist dieser ein Applikationssystem auf, das aus zwei Stützfolien (22a, 22b), zwei Griffleisten (25, 26) sowie zwei Adhäsiven (24a, 24b) besteht. Die beiden Stützfolien sind derart auf den Polymerfilm aufgebracht, dass dieser bis auf einen stützfolienfreien Bereich (27) vollständig von dem Polymerfilm abgedeckt wird. Der abgedeckte Bereich des Polymerfilms beträgt etwa 96 % der Fläche der ersten Seite des Polymerfilms. In dieser Ausführung überfängt die erste Griffleiste (25), die mittels des ersten Adhäsivs (24b) auf der ersten Stützfolie (22b) aufgeklebt ist, sowohl den stützfolienfreien Bereich (27) als auch die zweite Griffleiste (26), die mittels des zweiten Adhäsivs (24a) auf der zweiten Stützfolie (22a) aufgeklebt ist. Die erste Griffleiste (25) weist zum Ergreifen durch den Benutzer eine als Hintergriffsmittel ausgebildete Grifffläche auf. Der äußere Teil der Grifffläche ragt seitlich als freie Grifffläche (251) über die zweite Griffleiste (26) hinaus. Diese zweite Griffleiste (26) weist in der dargestellten Ausführungsform keine als Hintergriffsmittel ausgebildete Grifffläche auf. Dies ist aber wie in Figuren 3 und 6 bei einem Kanülenpflaster dargestellt ebenfalls denkbar und vorteilhaft, um das Ergreifen der zweiten Griffleiste (26) zu erleichtern. Dadurch, dass nur eine Griffleiste greifbar und für den Benutzer sichtbar ist, ist eine Reihenfolge beim Entfernen der beiden Stützfolien vorgegeben.

Mit Figur 3 und Figur 6 ist ein weiterer Folienverband (30) wiedergegeben. Dieser Folienverband kann als Kanülen- oder Katheterpflaster verwendet werden. Der Folienverband weist eine rechteckige Grundform auf, deren kurze Seite parallel zur langen Seite eine Aussparung aufweist. Durch diese Aussparung erhält der Folienverband zwei voneinander unabhängige Bereiche, die über einen dritten Bereich miteinander verbunden sind und die bei der Fixierung, zum Beispiel einer Kanüle jeweils auf einer Seite der Kanüle auf einer Oberfläche fixiert werden können. Der Folienverband weist einen Polymerfilm (1), eine Acrylat-Klebeschicht (2) und eine die klebende Schicht abdeckende Abdeckschicht (3) auf. Auf der der klebenden Schicht abgewandten ersten Seite des Polymerfilms ist ein Applikationssystem angeordnet. Dieses Applikationssystem umfasst zwei Griffleisten (35, 36), die mittels dreier Adhäsive (34a, 34b, 34c) auf drei Stützfolien (32a, 32b, 32c) aufgebracht sind. Die erste Griffleiste (35) ist sowohl der ersten Stützfolie (32c) als auch der zweiten Stützfolie (32b) zugeordnet mithin daran angeformt. Hierdurch können diese beiden Stützfolien (32c, 32b) mit einem Griff entfernt werden. Die erste Griffleiste (35) überfängt sowohl die zentralen Bereiche des Polymerfilmes, die nicht durch Stützfolien abgedeckt sind (37a, 37b) als auch die zweite Griffleiste (36). Der äußere Teil der Grifffläche ragt seitlich als freie Grifffläche (351) über die zweite Griffleiste (26) hinaus, so dass auch hier eine Reihenfolge beim Entfernen derselben vorgegeben ist.

Die Stützfolien bedecken insgesamt eine Fläche des Polymerfilms, die 92% der Fläche der ersten Seite des Polymerfilms beträgt, da neben den zentralen Bereichen (37a, 37b), die nicht durch Stützfolien abgedeckt sind, auch in den beiden Randbereichen (38a, 38b) keine Stützfolie angeordnet ist. Diese Randbereiche können nach dem Entfernen der Abdeckschicht zur Erstfixierung verwendet werden. Mit Figur 6 ist zudem ein Aktivierungsmittel (39) zwischen den beiden Griffleisten wiedergegeben. Dieses Aktivierungsmittel (39) ist wie auch das Adhäsiv zur Befestigung der Griffleiste (36) auf der Stützfolie (32a) in Figur 3 nicht dargestellt. Dieses Aktivierungsmittel (39) ist ein doppelseitiges Klebeband, das zur ersten Griffleiste (35) eine höhere Klebkraft aufweist als zur zweiten Griffleiste (36). Damit wird bei der Entfernung der Stützfolien (32b, 32c) mittels der ersten Griffleiste (35) die darunter liegende zweite Griffleiste (36) aufgerichtet, bevor die Klebkraft zwischen der ersten Griffleiste (35) und dem Aktivierungsmittel (39) aufgehoben wird. Die zweite Griffleiste (36) ist somit im nächsten Schritt zur Entfernung der zweiten Stützfolie (32a) leichter greifbar.

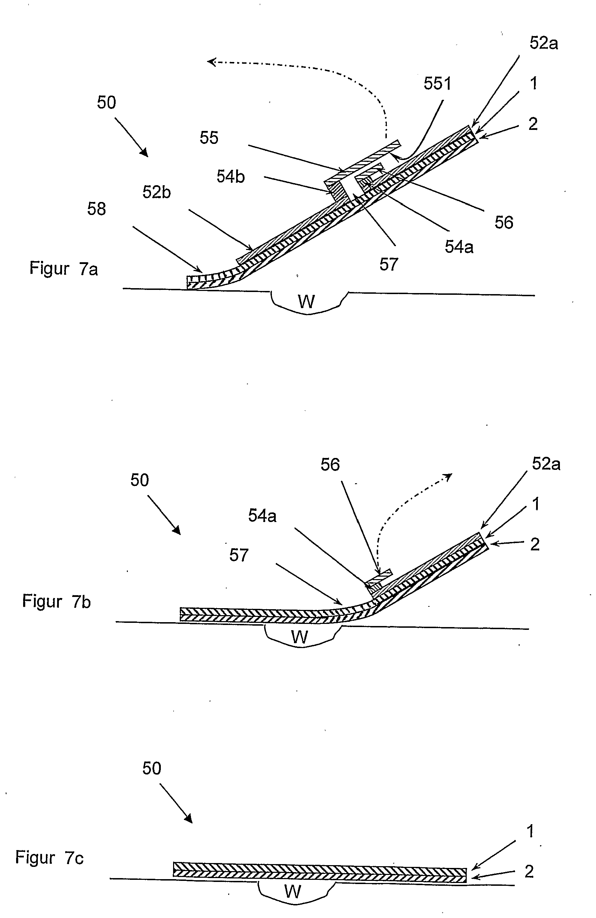

Bei der Anwendung eines erfindungsgemäßen Folienverbandes (10, 20, 30, 50) ist nun vorgesehen, zuerst die Abdeckschicht (3) von der Klebstoffschicht (2) zu entfernen. Wie in den Figuren 7a bis 7c gezeigt ist, kann bei einem Folienverband mit zwei stützfolienfreien Bereichen daraufhin, um einen Folienverband (30, 50) zum Beispiel oberhalb einer Wunde (W) zu applizieren, ein erster stützfolienfreier Randbereich (38a, 38b, 58) auf einem an die Wunde (W) angrenzenden Bereich fixiert werden. Durch die hohe Flexibilität des Polymerfilms im stützfolienfreien Bereich ist dies sehr gut möglich. Im weiteren Schritt kann der Anwender dann durch die vorgegebene Anordnung der Griffleisten (35, 36, 55, 56), die mittels adhäsiver Klebstoffe (34a, 34b, 34c, 54a, 54b) auf den jeweiligen Stützfolien aufgebracht sind, den zu applizierenden Polymerfilm (1) genau über der Wunde platzieren. Durch den zweiten stützfolienfreien Bereich (37a, 37b, 57) weist der Folienverband eine Art Gelenk auf, das eine faltenfreie Applikation gestattet. Die Stützfolien (32a, 32b, 32c, 52a, 52b) können nacheinander während der Applikation oder nacheinander nach erfolgter Applikation des Polymerfilms entfernt werden, wobei zunächst die erste Griffleiste (35, 55) über ihre freie Grifffläche (351, 551) ergriffen und damit zuerst die erste Stützfolie (32b, 32c, 52b) abgezogen werden kann.

### Ausführungsbeispiel 1:

Der Folienverband weist eine rechteckige Grundform mit einer Kantenlänge von 57 x 80 mm auf (Auflagefläche 45,6 cm²). Er umfasst einen transparenten Polyetherurethanfilm, der auf seiner in Gebrauch körperzugewandten Seite mit einem Hydrogel-Klebstoff auf Acrylat-Basis beschichtet ist. Der Klebstoff ist in einem vollflächigen Auftrag in einer Menge von ca. 35 g/ m² auf den ca. 25 µm dicken Polymerfilm (gemessen bei einem Prüfdruck von 0,5 kPa) aufgebracht. Der Polymerfilm weist zusammen mit dem Klebstoff eine Wasserdampfdurchlässigkeit von ca. 2600 g/ m²/ 24 Std. auf (gemessen nach DIN EN 13726, mit dem Unterschied, das nach 4 Std. Messzeit abgebrochen wurde und das ermittelte Ergebnis auf 24 Std. extrapoliert wird). Ein solcher Polymerfilm ist unter dem Handelsnamen Inspire 6200 bei der Fa. InteliCoat Technologies, Wrexham Industrial Estate, Wrexham LL13 9UF, UK, erhältlich. Die klebende Seite dieses Polymerfilms ist mit einem silikonisierten Abdeckpapier erhältlich unter dem Handelsnamen Separacon 980-60 bei der Fa. Maria Soell GmbH & Co. KG, Frankenstrasse 45, D-63667 Nidda-Eichelsdorf, abgedeckt. Auf der anderen, in Gebrauch körperabgewandten Seite des Polymerfilmes ist ein Applikationssystem angeordnet, das aus zwei Stützfolien besteht, auf denen jeweils eine Griffleiste angeordnet ist. Die Stützfolien sind wie in Figur 2 und Figur 5 dargestellt auf dem Polymerfilm angeordnet. In dem hier vorliegenden Folienverband ist jedoch zusätzlich ein Randbereich verwirklicht, der durch keine Stützfolie und keine Griffleiste abgedeckt ist. Dieser zusätzliche Randbereich ist auf der kurzen Seite des Rechtecks angeordnet und weist eine gleichförmige Breite von 5 mm auf. Die beiden Stützfolien sind gleich groß und weisen eine Kantenlänge von 57 x 36 mm auf (Auflagefläche: 2 x 20,5 cm² = 41,0 cm²). Der Abstand der beiden Folien beträgt in jedem Punkt ihrer zueinander parallel liegenden gleich langen Kanten ca. 3 mm. Damit ergibt sich insgesamt für beide Stützfolien eine Auflagefläche von 90 % bezogen auf Fläche der ersten Seite des Polymerfilms. Die Stützfolien sind aus einem 30 µm dicken Polyesterfilm gefertigt (gemessen bei einem Prüfdruck von 0,5 kPa). Auf jeder Stützfolie ist mittels eines Acrylat-Klebstoffes eine Griffleiste aufgeklebt. Die Griffleisten weisen im Querschnitt betrachtet eine Anordnung auf, wie sie mit Figur 6 wiedergegeben ist, wobei die erste mit Bezugszeichen (35) skizzierte Griffleiste eine Größe von 57 x 39 mm aufweist und über die gesamte Breite (57 mm) mit der zugehörigen Stützfolie verbunden ist. Die zweite mit Bezugszeichen (36) wiedergegeben Griffleiste weist eine Größe von 57 x 22 mm auf. Beide Griffleisten sind jeweils über einen 5 mm breiten adhäsiven Verbindungsstreifen mit der jeweiligen Stützfolie verbunden und sind aus einer 20 µm dicken transparenten Polyesterfolie gefertigt. Somit hat die erste Griffleiste eine Grifffläche, deren gleichförmige Breite 34 mm beträgt. Die Größe der Grifffläche der ersten Griffleiste beträgt 19,4 cm². Die gleichförmige Breite der Grifffläche der zweiten Griffleiste beträgt 17 mm. Somit beträgt die Größe der Grifffläche der zweiten Griffleiste 9,7 cm². Die gleichförmige Breite des über die zweite Griffleiste überstehenden Teils der ersten Grifffläche, also die Breite der freien Grifffläche der ersten Griffleiste bemisst sich mit 9 mm. Die Größe der freien Grifffläche beträgt somit 5,1 cm².

### Ausführungsbeispiel 2:

Der Folienverband weist eine rechteckige Grundform mit einer Kantenlänge von 57 x 80 mm auf (Auflagefläche 45,6 cm²). Er umfasst einen transparenten Polyetherurethanfilm, der auf seiner körperzugewandten Seite mit einem druckempfindlichen Haftklebstoff auf Acrylat-Basis beschichtet ist. Der Klebstoff ist in einem vollflächigen Auftrag in einer Menge von ca. 25 g/m² auf den ca. 30 µm dicken Polymerfilm (gemessen bei einem Prüfdruck von 0,5 kPa) aufgebracht. Der Polymerfilm weist zusammen mit dem Klebstoff eine Wasserdampfdurchlässigkeit von ca. 1200 g/ m²/ 24 Std. auf (gemessen nach DIN EN 13726). Ein solcher Polymerfilm ist unter dem Handelsnamen Inspire 1305 bei der Fa. InteliCoat Technologies, Wrexham Industrial Estate, Wrexham LL13 9UF, UK, erhältlich. Die klebende Seite dieses Polymerfilms ist mit einem silikonisierten Abdeckpapier erhältlich unter dem Handelsnamen Separacon 980-60 bei der Fa. Maria Soell GmbH & Co. KG, Frankenstrasse 45, D-63667 Nidda-Eichelsdorf, abgedeckt. Auf der anderen, in Gebrauch körperabgewandten Seite des Polymerfilmes ist ein Applikationssystem angeordnet, das aus zwei Stützfolien besteht, auf denen jeweils eine Griffleiste angeordnet ist. Die Stützfolien sind wie in Figur 2 und Figur 5 dargestellt auf dem Polymerfilm angeordnet. In dem hier vorliegenden Folienverband ist jedoch zusätzlich ein Randbereich verwirklicht, der durch keine Stützfolie und keine Griffleiste abgedeckt ist. Dieser zusätzliche Randbereich ist auf der kurzen Seite des Rechtecks angeordnet und weist eine gleichförmige Breite von 5 mm auf. Die beiden Stützfolien sind gleich groß und weisen eine Kantenlänge von 57 x 36 mm auf (Auflagefläche: 2 x 20,5 cm² = 41,0 cm²). Der Abstand der beiden Folien beträgt in jedem Punkt ihrer zueinander parallel liegenden gleich langen Kanten ca. 3 mm. Damit ergibt sich insgesamt für beide Stützfolien eine Auflagefläche von 90 % bezogen auf Fläche der ersten Seite des Polymerfilms. Die Stützfolien sind aus einem 30 µm dicken Polyesterfilm gefertigt (gemessen bei einem Prüfdruck von 0,5 kPa). Auf jeder Stützfolie ist mittels eines Acrylat-Klebstoffes eine Griffleiste aufgeklebt. Die Griffleisten weisen im Querschnitt betrachtet eine Anordnung auf, wie sie mit Figur 6 wiedergegeben ist, wobei die erste mit Bezugszeichen (35) skizzierte Griffleiste eine Größe von 57 x 39 mm aufweist und über die gesamte Breite (57 mm) mit der zugehörigen Stützfolie verbunden ist. Die zweite mit Bezugszeichen (36) wiedergegeben Griffleiste weist eine Größe von 57 x 22 mm auf. Beide Griffleisten sind jeweils über einen 5 mm breiten adhäsiven Verbindungsstreifen mit der jeweiligen Stützfolie verbunden und sind aus einer 20 µm dicken transparenten Polyesterfolie gefertigt. Somit hat die erste Griffleiste eine Grifffläche, deren gleichförmige Breite 34 mm beträgt. Die Größe der Grifffläche der ersten Griffleiste beträgt 19,4 cm². Die gleichförmige Breite der Grifffläche der zweiten Griffleiste beträgt 17 mm. Somit beträgt die Größe der Grifffläche der zweiten Griffleiste 9,7 cm². Die gleichförmige Breite des über die zweite Griffleiste überstehenden Teils der ersten Grifffläche, also die Breite der freien Grifffläche der ersten Griffleiste bemisst sich mit 9 mm. Die Größe der freien Grifffläche beträgt somit 5,1 cm².

Die Release-Eigenschaften der verwendeten Materialien des Ausführungsbeispiels 2 wurden an 60 x 80 mm großen Teststücken mittels der in DIN 53 530 beschriebenen Methode bestimmt. Die Prüfungen wurden bei einer Abzugsgeschwindigkeit von 300 mm/ min bestimmt. Demnach weist das Silikonpapier zum Polymerfilm eine Releasekraft von 0,77 N/ 25 mm auf wogegen der Stützfolie zum Polymerfilm eine Releasekraft von 0,09 N/ 25 mm aufweist. Damit sind die Release-Eigenschaften dieses Folienverbandes so eingestellt, dass die Abziehkraft, die notwendig ist, um eine Abdeckfolie von dem zu applizierenden Polymerfilm zu lösen, größer ist als die Abziehkraft, die notwendig ist, um die Stützfolie oder die Stützfolien von dem Polymerfilm zu trennen.

## Patentansprüche

1. Folienverband (10, 20, 30, 50) umfassend einen Polymerfilm (1) und ein Applikationssystem zur leichteren Handhabung des Folienverbandes, wobei das Applikationssystem auf einer ersten Seite des Polymerfilms (1) angeordnet ist und mindestens eine Stützfolie (12, 22a, 22b, 32a, 32b, 32c, 52a, 52b) umfasst, an der mindestens eine Griffleiste (16, 25, 26, 35, 36, 55, 56) angeformt ist, **dadurch gekennzeichnet, dass** das Applikationssystem zwei Stützfolien (22a, 22b, 52a, 52b) mit jeweils mindestens einer Griffleiste (25, 26, 55, 56) umfasst, wobei jede Griffleiste eine zusätzliche Materialkomponente ist und gleichzeitig mit mindestens einer Stützfolie von dem Polymerfilm entfernbar ist, und dass der Polymerfilm (1) mindestens einen ersten stützfolienfreien Bereich (17, 27, 37a, 37b, 38a, 38b, 57, 58) aufweist und eine Griffleiste (15, 25, 35, 55) den ersten stützfolienfreien Bereich (17, 27, 37a, 37b, 38a, 38b, 57, 58) zumindest abschnittsweise überfängt, wobei die Auflagefläche der Stützfolien insgesamt weniger als 94 % der Fläche der ersten Seite des zu applizierenden Polymerfilms entspricht.

2. Folienverband (10, 20, 30, 50) nach Anspruch 1 **dadurch gekennzeichnet, dass** eine Griffleiste (15, 25, 35, 55) den ersten stützfolienfreien Bereich (17, 27, 37a, 37b, 38a, 38b, 57, 58) vollständig überfängt.

3. Folienverband (10, 20, 30, 50) nach mindestens einem der vorangegangenen Ansprüche **dadurch gekennzeichnet, dass** die Stützfolien (22a, 22b, 32a, 32b, 32c, 52a, 52b) in einer Ebene parallel zum Polymerfilm (1) angeordnet sind.

4. Folienverband (10, 20, 30, 50) nach mindestens einem der vorangegangenen Ansprüche **dadurch gekennzeichnet, dass** der erste stützfolienfreie Bereich (27, 37a, 37b, 57) zwischen einer ersten und einer zweiten Stützfolie (22a, 22b, 32a, 32b, 32c, 52a, 52b) angeordnet ist.

5. Folienverband (10, 20, 30, 50) nach mindestens einem der Ansprüche 1-3 **dadurch gekennzeichnet, dass** der erste stützfolienfreie Bereich (17, 38a, 38b, 58) einen Randbereich des Polymerfilms (1) bildet.

6. Folienverband (10, 20, 30, 50) nach mindestens einem der vorgenannten Ansprüche **dadurch gekennzeichnet, dass** der Polymerfilm (1) mindestens einen zweiten stützfolienfreien Bereich (27, 37a, 37b, 38a, 38b, 57, 58) aufweist, der von dem ersten Bereich getrennt angeordnet ist.

7. Folienverband (10, 20, 30, 50) nach Anspruch 6 **dadurch gekennzeichnet, dass** der zweite stützfolienfreie Bereich einen Randbereich des Polymerfilms (1) bildet oder zwischen einer ersten und einer zweiten Stützfolie (22a, 22b, 32a, 32b, 32c, 52a, 52b) angeordnet ist.

8. Folienverband (10, 20, 30, 50) nach Anspruch 1 **dadurch gekennzeichnet, dass** eine der beiden Griffleisten (25, 26, 55, 56) die andere Griffleiste (25, 26, 55, 56) zumindest abschnittsweise, insbesondere vollständig überfängt.

9. Folienverband (10, 20, 30, 50) nach Anspruch 8 **dadurch gekennzeichnet, dass** die beiden Griffleisten (25, 26, 35, 36, 55, 56) unterschiedlich flexibel sind.

10. Folienverband (10, 20, 30, 50) nach mindestens einem der vorgenannten Ansprüche **dadurch gekennzeichnet, dass** die zweite Seite des Polymerfilms (1) mit einem Haftklebemitel, insbesondere vollflächig mit einem Haftklebemittel (2) beschichtet ist und das Haftklebemittel mit einer Abdeckfolie oder einem Abdeckpapier (3) abgedeckt ist.

11. Folienverband (10, 20, 30, 50) nach mindestens einem der vorgenannten Ansprüche **dadurch gekennzeichnet, dass** auf der zweiten Seite des Polymerfilms (1) ein Wundkissen aufgebracht ist.

12. Folienverband (10, 20, 30, 50) nach mindestens einem der vorgenannten Ansprüche **dadurch gekennzeichnet, dass** auf der zweiten Seite des Polymerfilms (1) eine die Wundheilung fördernde Schicht aufgebracht ist.

13. Folienverband (10, 20, 30, 40, 50) nach mindestens einem der vorgenannten Ansprüche **dadurch gekennzeichnet, dass** der Polymerfilm (1) ein Polyurethanfilm, Polyesterurethanfilm oder Polyetherurethanfilm ist.

## Claims

1. Film dressing (10, 20, 30, 50) comprising a polymer film (1) and an application system for easier handling of the film dressing, wherein the application system is arranged on a first side of the polymer film (1) and comprises at least one support film (12, 22a, 22b, 32a, 32b, 32c, 52a, 52b) onto which at least one gripping strip (16, 25, 26, 35, 36, 55, 56) is formed, **characterized in that** the application system comprises two support films (22a, 22b, 52a, 52b) each having at least one gripping strip (25, 26, 55, 56), wherein each gripping strip is an additional material component and is removable from the polymer film simultaneously with at least one support film and **in that** the polymer film (1) has at least one first support-film-free region (17, 27, 37a, 37b, 38a, 38b, 57, 58) and a gripping strip (15, 25, 35, 55) overlaps the first support-film-free region (17, 27, 37a, 37b, 38a, 38b, 57, 58) at least in sections, wherein the contact area of the support films altogether corresponds to less than 94% of the area of the first side of the polymer film to be applied.

2. Film dressing (10, 20, 30, 50) according to Claim 1, **characterized in that** a gripping strip (15, 25, 35, 55) overlaps the first support-film-free region (17, 27, 37a, 37b, 38a, 38b, 57, 58) completely.

3. Film dressing (10, 20, 30, 50) according to at least one of the preceding claims, **characterized in that** the support films (22a, 22b, 32a, 32b, 32c, 52a, 52b) are arranged in a plane parallel to the polymer film (1).

4. Film dressing (10, 20, 30, 50) according to at least one of the preceding claims, **characterized in that** the first support-film-free region (27, 37a, 37b, 57) is disposed between a first and a second support film (22a, 22b, 32a, 32b, 32c, 52a, 52b).

5. Film dressing (10, 20, 30, 50) according to at least one of Claims 1-3, **characterized in that** the first support-film-free region (17, 38a, 38b, 58) forms an edge region of the polymer film (1).

6. Film dressing (10, 20, 30, 50) according to at least one of the preceding claims, **characterized in that** the polymer film (1) has at least a second support-film-free region (27, 37a, 37b, 38a, 38b, 57, 58) which is disposed separately from the first region.

7. Film dressing (10, 20, 30, 50) according to Claim 6, **characterized in that** the second support-film-free region forms an edge region of the polymer film (1) or is disposed between a first and a second support film (22a, 22b, 32a, 32b, 32c, 52a, 52b).

8. Film dressing (10, 20, 30, 50) according to Claim 1, **characterized in that** one of the two gripping strips (25, 26, 55, 56) overlaps the other gripping strip (25, 26, 55, 56) at least in sections, in particular completely.

9. Film dressing (10, 20, 30, 50) according to Claim 8, **characterized in that** the two gripping strips (25, 26, 35, 36, 55, 56) have different degrees of flexibility.

10. Film dressing (10, 20, 30, 50) according to at least one of the preceding claims, **characterized in that** the second side of the polymer film (1) has a covering of pressure-sensitive adhesive, in particular an allover covering of pressure-sensitive adhesive (2), and the pressure-sensitive adhesive is covered with a covering film or a covering paper (3).

11. Film dressing (10, 20, 30, 50) according to at least one of the preceding claims, **characterized in that** the second side of the polymer film (1) has a wound pad applied to it.

12. Film dressing (10, 20, 30, 50) according to at least one of the preceding claims, **characterized in that** the second side of the polymer film (1) has a layer which promotes wound healing applied to it.

13. Film dressing (10, 20, 30, 40, 50) according to at least one of the preceding claims, **characterized in that** the polymer film (1) is a polyurethane film, polyester urethane film or polyether urethane film.

## Revendications

1. Pansement film (10, 20, 30, 50) comprenant un film polymère (1) et un système d'application pour faciliter la manipulation du pansement film, le système d'application étant disposé sur une première face du film polymère (1) et comprenant au moins une feuille d'appui (12, 22a, 22b, 32a, 32b, 32c, 52a, 52b), contre laquelle est rapportée au moins une ailette de préhension (16, 25, 26, 35, 36, 55, 56), **caractérisé en ce que** le système d'application comprend deux feuilles d'appui (22a, 22b, 52a, 52b) ayant chacune au moins une ailette de préhension (25, 26, 55, 56), chaque ailette de préhension étant un composant matériel supplémentaire et pouvant être enlevée du film polymère simultanément avec au moins une feuille d'appui, et **en ce que** le film polymère (1) comprend au moins une première zone (17, 27, 37a, 37b, 38a, 38b, 57, 58) sans feuille d'appui et une ailette de préhension (15, 25, 35, 55) recouvre au moins en partie la première zone (17, 27, 37a, 37b, 38a, 38b, 57, 58) sans feuille d'appui, la surface d'appui de la feuille d'appui représentant dans l'ensemble moins de 94 % de la surface de la première face du film polymère à appliquer.

2. Pansement film (10, 20, 30, 50) selon la revendication 1, **caractérisé en ce qu'**une ailette de préhension (15, 25, 35, 55) recouvre complètement la première zone (17, 27, 37a, 37b, 38a, 38b, 57, 58) sans feuille d'appui.

3. Pansement film (10, 20, 30, 50) selon au moins l'une des revendications précédentes, **caractérisé en ce que** les feuilles d'appui (22a, 22b, 32a, 32b, 52a, 52b) sont disposées dans un plan parallèlement au film polymère (1).

4. Pansement film (10, 20, 30, 50) selon au moins l'une des revendications précédentes, **caractérisé en ce que** la première zone (27, 37a, 37b, 57) sans feuille d'appui est disposée entre une première et une deuxième feuilles d'appui (22a, 22b, 32a, 32b, 32c, 52a, 52b).

5. Pansement film (10, 20, 30, 50) selon au moins l'une des revendications 1 à 3, **caractérisé en ce que** la première zone (17, 38a, 38b, 58) sans feuille d'appui forme une zone de bordure du film polymère (1).

6. Pansement film (10, 20, 30, 50) selon au moins l'une des revendications précédentes, **caractérisé en ce que** le film polymère (1) comprend au moins une deuxième zone (27, 37a, 37b, 38a, 38b, 57, 58) sans feuille d'appui, qui est disposée séparément de la première zone.

7. Pansement film (10, 20, 30, 50) selon la revendication 6, **caractérisé en ce que** la deuxième zone sans feuille d'appui forme une zone de bordure du film polymère (1) ou est disposée entre une première et une deuxième feuille d'appui (22a, 22b, 32a, 32b, 32c, 52a, 52b).

8. Pansement film (10, 20, 30, 50) selon la revendication 1, **caractérisé en ce que** l'une des deux ailettes de préhension (25, 26, 55, 56) recouvre au moins par endroits, en particulier complètement, l'autre ailette de préhension (25, 26, 55, 56).

9. Pansement film (10, 20, 30, 50) selon la revendication 8, **caractérisé en ce que** les deux ailettes de préhension (25, 26, 35, 36, 55, 56) ont des flexibilités différentes.

10. Pansement film (10, 20, 30, 50) selon au moins l'une des revendications précédentes, **caractérisé en ce que** la deuxième face du film polymère (1) est revêtue d'un adhésif de contact, en particulier en pleine surface d'un adhésif de contact (2), l'adhésif de contact étant recouvert d'une feuille de recouvrement ou d'un papier de recouvrement (3).

11. Pansement film (10, 20, 30, 50) selon au moins l'une quelconque des revendications précédentes, **caractérisé en ce qu'**un coussinet-pansement est appliqué sur la deuxième face du film polymère (1).

12. Pansement film (10, 20, 30, 50) selon au moins l'une quelconque des revendications précédentes, **caractérisé en ce qu'**une couche favorisant la cicatrisation est appliquée sur la deuxième face du film polymère (1).

13. Pansement film (10, 20, 30, 40, 50) selon au moins l'une des revendications précédentes, **caractérisé en ce que** le film polymère (1) est un film de polyuréthanne, un film de polyester-uréthanne ou un film de polyéther-uréthanne.
